# EUROPEAN PATENT SPECIFICATION

(11) **EP 2 750 511 B1**
(45) Date of publication and mention of the grant of the patent: **12.12.2018**
(21) Application number: 12759165.9
(22) Date of filing: 31.08.2012
(51) Int. Cl.: A23C 9/127, A23C 9/14, A23C 9/142

(54) **PRODUCT AND PROCESS FOR ITS MANUFACTURE**
PRODUKT UND VERFAHREN ZU SEINER HERSTELLUNG
PRODUIT ET PROCÉDÉ POUR SA FABRICATION

(30) Priority: 31.08.2011 FI 20115851
(43) Date of publication of application: 09.07.2014
(73) Proprietor: Valio Ltd, 00370 Helsinki (FI)
(72) Inventor: RAJAKARI, Kirsi, FI-00370 Helsinki (FI); MYLLÄRINEN, Päivi, FI-00370 Helsinki (FI)
(74) Representative: Kolster Oy Ab
(86) International application number: PCT/FI2012/050848
(87) International publication number: WO 2013/030461

(56) References cited:
- EP-A1- 1 464 230
- EP-A1- 1 464 230
- WO-A1-93/19610
- WO-A1-2007/060288
- WO-A1-2008/000913
- WO-A2-2010/089381
- US-B1- 6 416 797
- Lucey: "Cultured dairy products: an overview of their gelation and texture properties", International Journal of Dairy Technology, 1 January 2004 (2004-01-01), pages 77-84, XP55040280, Retrieved from the Internet: URL:http://onlinelibrary.wiley.com/store/1 0.1111/j.1471-0307.2004.00142.x/asset/j.14 71-0307.2004.00142.x.pdf?v=1&t=h81gk1ou&s= cb785d27a0008f06683bd628d106d88d788b618b [retrieved on 2012-10-08]
- LOPEZ C ET AL: "Fat globules selected from whole milk according to their size: Different compositions and structure of the biomembrane, revealing sphingomyelin-rich domains", FOOD CHEMISTRY, ELSEVIER LTD, NL, vol. 125, no. 2, 15 March 2011 (2011-03-15), pages 355-368, XP027454030, ISSN: 0308-8146, DOI: 10.1016/J.FOODCHEM.2010.09.005 [retrieved on 2010-09-07]

## Description

### Field of the invention

The invention relates to a process for the manufacture of a soured milk product by means of physical modification of the milk raw material and a crosslinking enzyme that strengthens the texture. The invention also relates to a milk product that contains physically modified milk raw material fat globules and has been treated with a crosslinking enzyme.

### Background of the invention

In the preparation of sour milk products, it is typical that starter is added to homogenized, heat-treated milk, which, as it sours the milk, provides it with the texture and taste properties typical of the product.

Conventionally all the necessary raw materials and ingredients, such as sweetener, flavourings and texturizers, are added to the milk, and the milk mixture is then typically homogenized and pasteurized prior to souring. The homogenization is conventionally done by using either one- or two-phase homogenizers, and the typical homogenization conditions are a temperature of 55 to 80 °C, more typically of 65 to 70°C and a pressure of 100 to 250 bar, more typically of 150 to 200 bar. The milk mixture is soured or the acidity is adjusted to a pH value specific for each product in some other manner. After this, the texture is optionally broken and cooled to a packaging temperature, the necessary flavourings (e.g. jam) are added and the product is packaged.

The effects of milk high-pressure homogenization on the particle size, water retention, syneresis, and texture of yogurts at a pressure of 300 to 3500 bar have been extensively studied in the last few years (Lanciotti, R. et al., Food Microbiology, 21 (2004) 753 - 760; Ciron, C.I.E. et al., Int. Dairy J. 20 (2010) 314 - 320). The high mechanical forces / shear forces directed to milk in high-pressure homogenization help reduce the size of the fat globules in milk. It is known that the particle size of an emulsion can be reduced by ultrasound treatment or power ultrasound treatment.

Basic information exists on the advantageous effects of high-pressure homogenization and ultrasound technology of milk raw material on the consistency, such as viscosity and structural (gel) hardness, of a sour milk product and on the reduction in syneresis. However, the problem in the preparation of low-fat products in particular is the poor resistance to downstream processing, such as modification and texture breakage.

Publication EP1464230 describes the preparation of desserts and soured products at a pressure of 400 to 2000 bar from a homogenized milk-based emulsion.

Publication US 6416797, Kraft Foods, describes a preparation process for a cream cheese (Philadelphia cream cheese), in which transglutaminase and starter are added to the raw material liquid, it is soured to a pH value of 4.5, and then the soured milk-based emulsion mixture is homogenized, if desired, at a high pressure (approximately 690 bar, 10 000 psi) to modify the soured product mixture. In the process described in the publication, the downstream processing of the cream cheese, or unripened cheese, becomes easier.

Conventionally, increasing the dry content of milk by evaporating, concentrating and/or adding powder to milk, for instance, and decreasing the dry content of milk, among other things, have been used in adjusting the texture of sour milk products. The protein content of raw material milk affects the texture of the final product, and the texture of a milk product can be modified as necessary by increasing or decreasing the protein content. For instance, the protein content of yogurt milk can be increased by evaporation or by adding protein powders, such as milk, whey and casein protein powder, therein. Protein supplements based on non-milk proteins are also useful. Alternatively, the thinning of the texture can be done by adding milk permeate, cheese whey, acid whey, such as quark and/or cottage cheese whey, or water to the raw material milk.

It is also known that by using a crosslinking enzyme in sour milk products, the texture can be hardened and thickened and modified to be more fine texture, and the separation of whey reduced. To minimize structural problems, it is well known in the art to add to the protein source a crosslinking enzyme that modifies the texture. The processes conventionally use milk homogenized at low pressures (100-250 bar) for the preparation of soured milk products. A problem then arises that the processes and their control are further complicated and become more difficult as more preparation steps are added. Thus, simple product formulations and cost-effective preparation processes are needed to control the problems, such as post-souring and structural problems like a powdery texture, caused by the generally known processes to the products.

In general, problems with the known processes include the alteration of the organoleptic properties of sour milk products and their poor shelf-life during storage. Syneresis, separation of whey and structural problems occur in the products. Problems related to downstream processing, such as mass modification and texture breakage, as well as to shelf-life are especially emphasized in low-fat sour milk products.

WO2007060288, WO2010089381 and WO2008000913 disclose processes for preparing textured sour milk products in which a cross-linking enzyme, transglutaminase, is used. These processes employ neither high-pressure homogenization nor microfiltration.

### Brief description of the invention

The invention relates to a process for the manufacture of a sour milk product by means of physical modification of the milk raw material and/or the fat portion thereof, and a crosslinking enzyme that strengthens the texture. Therefore, the invention provides a process that combines the physical modification of fat globules and treatment with a crosslinking enzyme in order to prepare soured milk products and to modify and stabilize their texture. The process is simple, economical, and industrially applicable on large scale, and it does not cause additional costs. Further, the process of the invention provides significant savings when the amounts of the components in the milk raw material, such as fat content and/or protein content, may be reduced without affecting the texture and properties of the product being prepared. The possibility of affecting the amounts of different milk raw material components is important, because the dairy industry currently uses component manufacturing. The invention also relates to a milk product that contains physically modified milk raw material fat globules and has been treated with a crosslinking enzyme. Thus, the invention relates to a soured milk product which is optimal in relation to the different components of the milk raw material and withstands well downstream processing steps of preparation, such as mass modification and texture breaking, and whose structural properties also keep in storage.

It is very challenging to achieve a soured milk product which contains physically modified milk raw material fat globules, is completely flawless in taste and texture, meets consumer expectations, and withstands both the demanding downstream processing steps of sour milk product preparation, such as mass modification and texture breaking, and storage, and is made in an economical and simple manner. Adjusting the concentrations of raw material components, such as reducing fat, in a product is challenging. It was surprisingly found that a soured product of a desired type that is clearly thicker in texture was obtained by physically modifying the milk raw material and treating it with a crosslinking enzyme during souring.

With the process of the invention, it is possible to improve the structural/texture properties of the product being prepared by physically modifying the particle size, size distribution, composition and condition of the fat globules in the milk raw material.

The object of the invention is achieved with a product and process that are characterized by what is stated in the independent claims. Preferred embodiments of the invention are disclosed in the dependent claims. Working examples that do not fall within the scope of the independent claims are to be considered as illustrative of or comparative to the claimed invention, and do not form part of the invention as claimed.

### Brief description of the figures

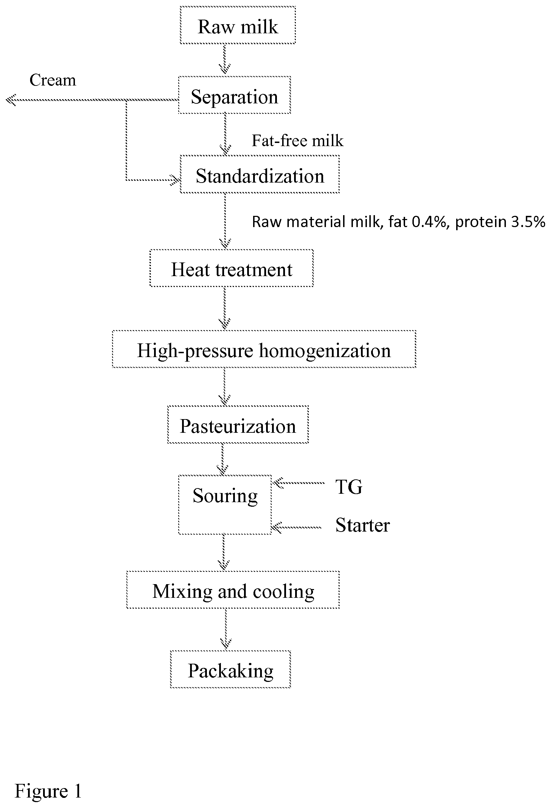

Figure 1 is a flow diagram showing an embodiment of the preparation process of a soured milk product according to the invention, when preparing a low-fat yogurt with reduced protein content. In the process milk raw material with standardized fat and protein content (0.4 % fat, 3.5 % protein) was physically treated by high-pressure homogenization at a pressure of 400 bar.

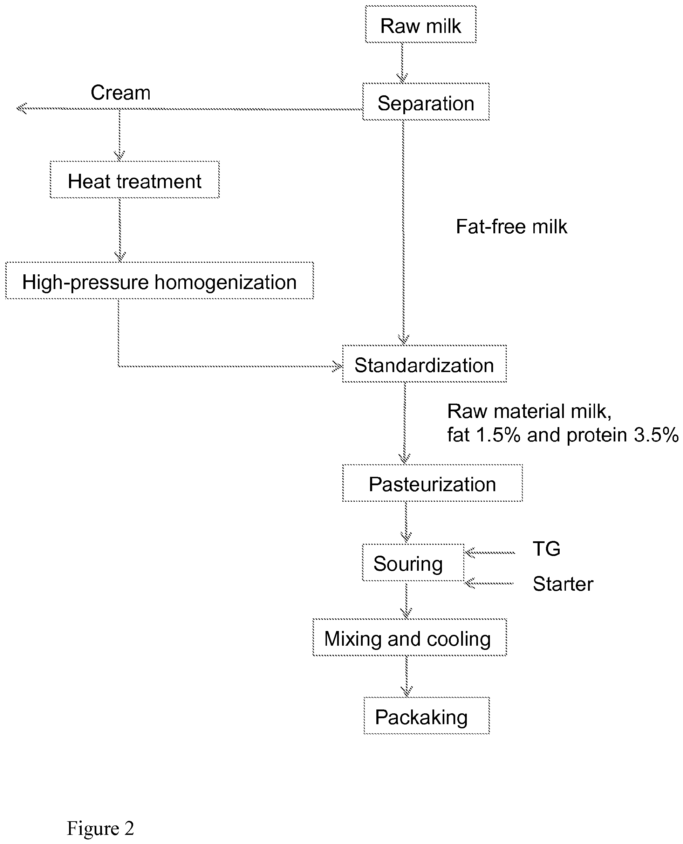

Figure 2 is a flow diagram showing an embodiment of the preparation process of a soured milk product according to the invention, when preparing a low-fat yogurt. In the process the fat portion was physically treated by high-pressure homogenization at a pressure of 1000 bar.

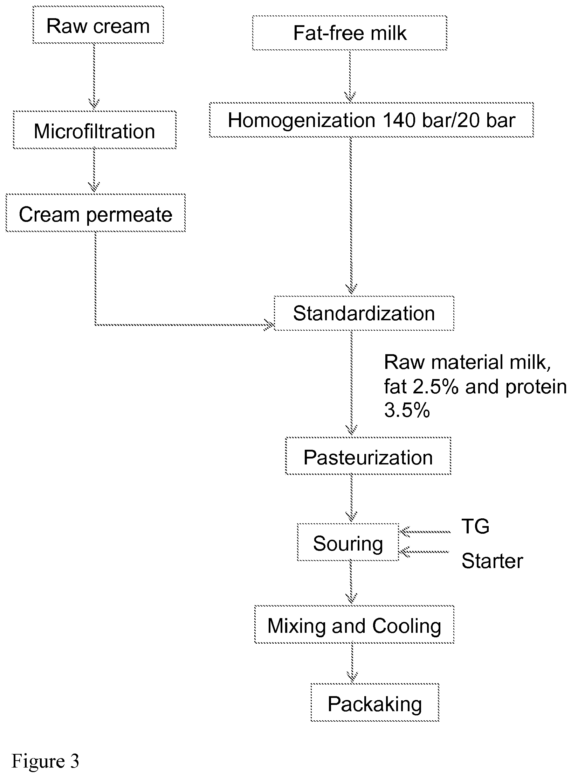

Figure 3 is a flow diagram showing an embodiment of the preparation process of a soured milk product according to the invention, where fat is physically modified by means of microfiltration.
Figure 4 shows the particle size distribution of non-homogenized standardized yogurt milk having a fat content of 1%, measured by particle size analyzer (Malvern).
Figure 5 shows the particle size distribution of homogenized (220 bar/40 bar), standardized yogurt milk having a fat content of 1%, measured by particle size analyzer (Malvern).
Figure 6 shows the particle size distribution of high-pressure homogenized (400 bar/70 bar), standardized yogurt milk, fat content 1%, measured by particle size analyzer (Malvern).

### Detailed description of the invention

The invention relates to a soured milk product that contains physically modified milk raw material fat globules. In addition, the invention relates to a soured milk product that contains physically modified milk raw material fat globules, i.e. micro/nano particles of milk raw material fat, and has been produced by means of a crosslinking enzyme, and to processes for the preparation of such a product. With the invention, it is possible to optimize a product in relation to the different components of the milk raw material and still maintain the texture and stability of the structure/texture during storage.

It was surprisingly found that by physically modifying milk raw material and treating it with a crosslinking enzyme during souring, a sour milk product is obtained that is clearly thicker in texture than a corresponding product produed of high-pressure homogenized milk raw material. Small fat globules created as a result of the physical modification significantly increase the viscosity of a sour milk product, such as yogurt, and the formed small fat globules also prevent syneresis more efficiently than bigger unmodified fat globules and their clusters. Without wishing to be bound by a theory, it can be assumed that the small fat globules are coated with casein and whey protein and the cross-linking enzyme, such as transglutaminase, crosslinks the small fat globules with the matrix. The proteins inherent to the membrane structures of the fat globules are crosslinked. Possibly due to the small fat globules, in which case the structure has more surface and/or less void volume, the caseins and whey proteins get close to each other. Their crosslinking is then enhanced, as the activity of the transglutaminase becomes easier.

In addition, it was surprisingly found that the same effect can be produced by physically modifying only the fat portion of the milk raw material and combining it with non-homogenized fat-free milk or fat-free milk homogenized conventionally at low pressures, and treating the thus obtained milk with a crosslinking enzyme.

Further, it was surprisingly found that the same effect is produced by using, in the preparation of a soured milk product, native small fat globules originating from milk or some other suitable fat source, such as soy milk or coconut milk, and/or physically, with microfiltration, for instance, modified small fat globules that are combined with non-homogenized fat-free milk or fat-free milk homogenized conventionally at low pressures, and treating the thus obtained milk with a crosslinking enzyme.

Thus, according to an embodiment of the invention, the milk raw material is physically modified and treated with a crosslinking enzyme. According to a second embodiment of the invention, the fat portion of the milk raw material is modified physically and combined with non-homogenized fat-free milk or fat-free milk homogenized conventionally at low pressures, and the thus obtained milk or milk raw material is treated with a crosslinking enzyme. According to a third embodiment of the invention, native and/or physically modified small fat globules are combined with non-homogenized fat-free milk or fat-free milk homogenized conventionally at low pressures, and the thus obtained milk or milk raw material is treated with a crosslinking enzyme.

The invention also relates to a process for the modification of the texture of soured milk products by means of physical modification of the milk raw material and a crosslinking enzyme that strengthens the texture.

It was surprisingly found that by means of the processes according to the invention, it is possible either to prepare a clearly thicker sour milk product or to reduce the protein content and/or fat content of the product while the texture of the final product remains the same. This means either a significant improvement in the quality of the product or significant savings in the raw materials / ingredients. The processes can be applied to the preparation of yogurt, drinkable yogurt, set-type yogurt, viili, and fermented milk, in particular.

The processes of the invention are thus suitable for the preparation of soured products having excellent taste and texture. By means of the invention, it is also possible to reduce the fat content of the product and/or maximize its protein content. The product of the invention is characterized in that it withstands the mass modification and texture breaking typical of sour milk products during preparation, as well as the storage of the product.

In connection with the present invention, physical modification of the milk raw material refers to a procedure that is done through high mechanical forces / shear forces directed to the raw milk material or with separation techniques or a combination of these, with which especially the particle size and particle size distribution, but also composition and condition of the milk raw material fat globules are changed.

In connection with the present invention, the term "a physically modified fat globule"/"physically modified fat globules" refers to a fat globule/fat globules having a size of from 20 nm to 1 µm, and more preferably from 100 nm to 1 µm. In addition, the fat globule/fat globules have an average particle size, or medium size less than 1 µm, preferably from 200 to 500 nm, and more preferably from 300 to 400 nm.

Industrial-scale mixers having a sufficient energy density to provide high mechanical forces / shear forces and capable of producing fat globules with a particle size of less than 10 µm include homogenizers (less than 4 µm), high-pressure homogenizers (less than 2 µm), micro-fluidizers (less than 1 µm), rotor-stator systems (Ultra Turrax; particle size of 1 to 25 µm), and colloid mills (particle size of 1 to 25 µm). The size, size distribution, composition, and condition of the milk raw material fat globules can also be physically modified by extrusion, ultrasound treatment (low frequency, power ultrasound), optical technology (Laser, Light Amplification by Stimulated Emission of Radiation), HPP technology (high pressure processing), atomizer (spray drying), non-thermal pulsed electric field (PEF) technology, and a combination of different techniques. MTS (manothermosonication) technology combines treatment with ultrasound under pressure and a temperature lower than the pasteurization temperature. Membrane techniques, such as microfiltration and gravitation, as well as combinations of different techniques can be used as the separation technique.

According to an embodiment, the physical modification is done by high-pressure homogenization at a temperature of 40 to 95°C, preferably 60 to 70°C, and a pressure of 250 to 1000 bar, preferably 250 to 500 bar. In an embodiment, the pressure of the high-pressure homogenization is 400 bar. In another embodiment of the invention, high-pressure homogenization is done in two steps. In a second embodiment of the invention, the high-pressure homogenization is done in two steps in such a manner that in the first step, the temperature is 40 to 95°C and the pressure is 250 to 1000 bar, and in the second step, the temperature is 40 to 95°C and the pressure less than 1000 bar. In the first step, the size of fat globules is decreased, and in the second step, any fat globules that have attached to each other are separated into individual globules. In the second step, the homogenization conditions may correspond to those of high-pressure homogenization i.e., having a temperature of 40 to 95°C, preferably 60 to 70°C, and a pressure of 250 to 1000 bar, preferably 250 to 500 bar or be either those used in conventional homogenization i.e., having a temperature of 65 to 70°C and a pressure of 150 to 200 bar or even lower than them. In one embodiment, in the first step, the pressure is 400 bar, and in the second step, the pressure 70 bar.

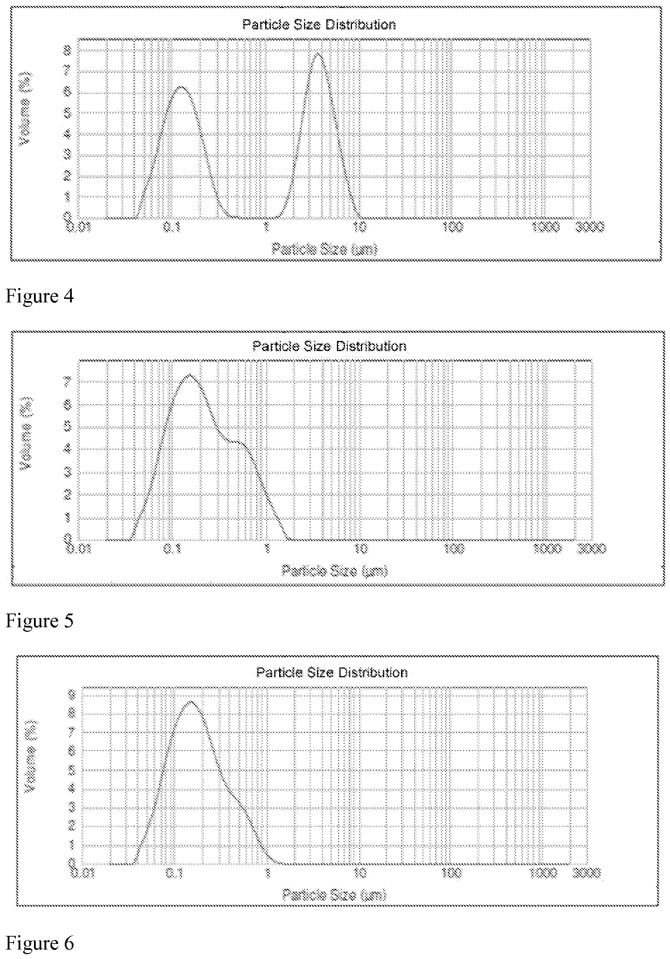

As can be seen from Figure 4, in non-homogenized milk the fat globules are within the size area from 1 µm to 10 µm and caseins are within the size area from 0.04 µm to 0.4 µm. The traditional homogenization (100 - 250 bar) reduces the size of fat globules to below 2 µm (see Figure 5). The high pressure homogenisation (250 - 1000 bar) reduces the fat globule size even further i.e., to below 1 µm (see Figure 6).

In addition to the particle sizes of the fat globules, the particle volumes of particles smaller than about 1 µm are affected by high-pressure homogenization. The particle volume share of fat globules having particle size for example less than about 1 µm, less than about 0.6 µm and less than about 0.3 µm, is higher after high-pressure homogenization than after conventional homogenization. High-pressure homogenization increases the volume share of fat globules having particle size less than about 1 µm, less than 0.6 µm and less than 0.3 µm by about 10 %, about 15 % and about 15 %, respectively, compared to the traditional homogenization. As can be seen from Table 1, the volume share of fat globules having particle size less than about 1 µm increases from about 91.5 % to 99.7 % when the homogenization pressure increases from 220 bar to 400 bar. Correspondingly, the volume share of fat globules having particle size less than about 0.6 µm increases from about 80.8 % to 94.8 % and the volume share of fat globules having particle size less than about 0.3 µm increases from about 65.2 % to 78.4 % when the homogenization pressure increases from 220 bar to 400 bar.

**Table 1.**

| Particle volume share% | | |
|---|---|---|
| Particle size µm | Homogenisation 220/40 bar | Homogenisation 400/70 bar |
| < 0.3 | 65.15 | 78.36 |
| < 0.6 | 80.75 | 94.80 |
| < 1.0 | 91.48 | 99.72 |

After the high-pressure homogenization, the fat globules could be thought to be acting like caseins in milk based products, such as yoghurts of the present invention, and this can be seen in thicker texture of the product and/or in reduced protein and/or fat content of the product while the texture of the product remains unchanged.

According to a second embodiment of the invention, physical modification is done by microfiltration. Microfiltration (MF) is a membrane filtration process, in which the average pore size of the used membranes is 0.05 to 10 µm. The components contained in a fluid to be separated are forced through the membrane by means of pressure.

In microfiltration, native fat globules divided into different size categories do not lose the original structure of their membrane, as occurs in homogenization, for instance. Homogenization under pressure affects the fat globules in such a manner that it breaks large fat globules into smaller ones, whereby part of the membrane of the globules is replaced by casein proteins. This changes the properties of the fat globules in such a manner that they are less susceptible to the oxidative spoilage effects of fats, for example. The fat globules that have been reduced in size by homogenization and coated in casein act to some extent like casein.

According to another embodiment of the invention, the physical modification is done by high-pressure homogenization and microfiltration in any order. In this embodiment, the milk raw material can for instance first be microfiltered and then the retentate obtained from microfiltration is high-pressure homogenized and combined with the microfiltration permeate for further processing.

In milk, fat is present in globules having a diameter that varies between 0.1 and 15 µm. The average fat content of raw milk is 4.5%. Approximate 80% of the fat globules in milk have a diameter of less than 1 µm in size, but the total fat content has less than 10% of small fat globules. The average diameter of a fat globule is approximately 4 µm. It has been noticed that large (over 2 µm) fat globules cluster more easily than the small (less than 2 µm) and are more susceptible to lipolysis. Large globules also bind less water.

In this patent application, the term "physical modification of milk raw material" covers not only the above-mentioned processes, but also modification done by means of enzymes (fat affecting enzymes, phospholipases, lipases and the like).

The terms "mechanically modified fat globules", "physically modified fat globules", micro-particles", "nano-particles" and "enzymatically modified fat globules" are used in parallel.

In addition, the terms "fat globule of milk raw material" and "microparticle/nano-particle of milk raw material fat" are used in parallel. The term "fat globules of milk raw material" refers to the micro- and/or nano-particles or fatty acid chains or parts separated from the fat globules of milk raw material fat.

According to an embodiment of the invention, the size of the fat globules obtained as a result of the physical modification is less than about 1 µm, preferably from about 20 nm to about 1 µm, and more preferably from about 100 nm to about 1 µm. According to a second embodiment of the invention, the average particle size, or medium size, of the fat globules obtained as a result of the physical modification is less than about 1 µm, preferably from about 200 to about 500 nm, and more preferably from about 300 to about 400 nm. According to a second embodiment of the invention, the size of the fat globules obtained as a result of the physical modification is less than about 1 µm, preferably from about 20 nm to about 1 µm, and more preferably from about 100 nm to about 1 µm, and the average particle size, or medium size, is less than about 1 µm, preferably from about 200 to about 500 nm, and more preferably from about 300 to about 400 nm.

The milk raw material may be milk, whey and combinations of milk and whey as such or as concentrate. The milk raw material may be milk as such obtained from an animal, such as a cow, sheep, goat, camel, mare or any other animal that produces milk suitable for human consumption, or milk that is pre-processed as desired. The milk raw material may be, for instance, whole milk, cream, low-fat or fat-free milk, low-lactose or lactose-free milk, colostrum, ultrafiltered milk, diafiltered milk, microfiltered milk, or milk reconstituted from milk powder, organic milk or a combination of these. According to an embodiment of the invention the fat content of the milk raw material is 0.4 to 2%. The milk raw material is preferably low-fat milk (fat content 0.5 to 1.5%) standardized with cream (30 to 50%) or semi-skimmed milk (1.5 to 4%) or fat-free milk (fat content less than 0.5%) standardized with whole milk (over 4%).

The milk raw material may be supplemented by ingredients generally used in producing milk products and/or whey and milk protein fractions, such as milk protein, whey protein, casein, whey and milk protein fractions, milk salt, α-lactalbumin, peptides, amino acids, e.g. lycine, as such or in different combinations and amounts depending on the product being prepared. The milk raw material may be supplemented by vegetable fat, such as rapeseed oil, corn oil, sunflower oil, berry oils as such or in different combinations and amounts depending on the product being prepared. Possible other optional supplements/components are omega-3 fatty acids, antioxidants and/or water-soluble or fat-soluble vitamins, cholesterol content-affecting sterols and their esters, and satiation-increasing compounds or compositions, such as food fat compositions having an oil-in-water emulsion structure and milk salts. In the process of the invention, these optional components may be used as such or in different combinations and amounts depending on the product being prepared. The whey and milk protein fractions may be produced by ultra- or nanofiltration (NF retentate), for instance.

The present invention provides a new solution for avoiding structural and quality defects that have shown to cause problems in the preparation of sour milk products by using a process that is characterized by treating milk raw material containing physically modified fat globules with a crosslinking enzyme and souring it.

According to the invention, the milk raw material is physically modified and treated with a crosslinking enzyme. According to this embodiment, the milk raw material containing physically modified fat globules is obtained by physically modifying the particle size, size distribution, composition, and condition of the fat globules. Physical modification can be done by utilizing high-pressure homogenization and/or microfiltration. Thus, in an alternative within the scope of this embodiment, the milk raw material or part thereof is fractionated by microfiltration, and the permeate containing small fat globules is transferred to treatment performed with a crosslinking enzyme as such or together with a further high-pressure processed retentate. Between the physical modification of the milk raw material and the treatment with a crosslinking enzyme, other processing steps may be performed optionally as desired and/or required. Further, other processing steps may also be performed before the physical modification of the milk raw material. Similarly, other processing steps may be performed after the treatment with a crosslinking enzyme.

According to a second embodiment of the invention, the fat portion of the milk raw material is modified physically and combined with non-homogenized fat-free milk or fat-free milk homogenized conventionally at low pressures, and the thus obtained milk raw material mixture is treated with a crosslinking enzyme and soured. According to this embodiment, the milk raw material containing physically modified fat globules is obtained by physically modifying the fat portion of the milk raw material and by combining it with non-homogenized or conventionally homogenized fat-free milk. Physical modification can be done by utilizing high-pressure homogenization and/or microfiltration. Thus, in an alternative within the scope of this embodiment, the fat portion of the milk raw material is fractionated by microfiltration, and the permeate containing small fat globules is transferred to treatment performed with a crosslinking enzyme as such or together with a further high-pressure processed retentate. Between the preparation of the milk raw material mixture containing physically modified fat globules and the treating with a crosslinking enzyme, other processing steps, such as heat treatment(s), may optionally be performed as desired and/or required. Further, before the preparation of the milk raw material containing physically modified fat globules, it is possible to perform other processing steps known in the art, such as heat treatment, standardization, reconstitution, protease, filtration, separation and/or freezing. Similarly, other processing steps may be performed after the treatment with a crosslinking enzyme.

According to a third embodiment of the invention, native and/or physically modified small fat globules are combined with non-homogenized fat-free milk or fat-free milk homogenized conventionally at low pressures, and the thus obtained milk raw material mixture is treated with a crosslinking enzyme and soured. According to this embodiment, the milk raw material containing physically modified fat globules is obtained by combining native and/or physically modified fat globules with non-homogenized or conventionally homogenized fat-free milk. Within the scope of this embodiment, the size of the native fat globules or fat globules obtained as a result of the physical modification is from 20 nm to 1 µm, and more preferably from 100 nm to 1 µm. According to an embodiment, the average particle size, or medium size, of the fat globules obtained as a result of the physical modification is less than about 1 µm, preferably from 200 to 500 nm, and more preferably from 300 to 400 nm. According to another embodiment, the size of the fat globules obtained as a result of the physical modification is from 20 nm to 1 µm, and more preferably from 100 nm to 1 µm, and the average particle size, or medium size, is less than 1 µm, preferably from 200 to 500 nm, and more preferably from 300 to 400 nm.

Between the preparation of the milk raw material mixture containing physically modified and/or native fat globules and the treatment with a cross-linking enzyme, other processing steps, such as heat treatment(s), may optionally be performed as desired and/or required. Further, before the preparation of the milk raw material mixture containing native and/or physically modified fat globules, it is possible to perform other processing steps known in the art. Similarly, other processing steps may be performed after the treatment with a crosslinking enzyme.

According to an embodiment of the invention, the process for the preparation of a sour milk product comprises
- the physical modification of milk raw material,
- treating with a crosslinking enzyme, and
- souring, and
- optionally the packaging of the product.

According to a second embodiment of the invention, the process for the preparation of a sour milk product comprises
- the physical modification of the fat portion of the milk raw material and its combination with non-homogenized fat-free milk or fat-free milk homogenized conventionally at low pressures
- treating with a crosslinking enzyme, and
- souring, and
- optionally the packaging of the product.

According to yet another embodiment of the invention, the process for the preparation of a sour milk product comprises
- the combination of native and/or physically modified fat globules with non-homogenized fat-free milk or fat-free milk homogenized conventionally at low pressures
- treating with a crosslinking enzyme,
- souring, and
- optionally the packaging of the product.

In the process of the invention, souring may be done before cross-linking enzyme treatment, simultaneously with the crosslinking enzyme treatment or only after the crosslinking enzyme treatment. In the process of the invention, the crosslinking enzyme treatment may be done simultaneously with souring, before souring, or only after souring. Thus, according to an embodiment of the invention, the process comprises the following steps:
- physically modifying the particle size, size distribution, composition, and condition of the fat globules of the milk raw material,
- souring,
- treating with a crosslinking enzyme,
- optionally packaging the product.

According to a second embodiment of the invention, the process comprises the following steps:
- physically modifying the particle size, size distribution, composition, and condition of the fat globules of the milk raw material,
- souring and treating with a crosslinking enzyme,
- optionally packaging the product.

Further according to yet another embodiment of the invention, the process comprises the following steps:
- physically modifying the particle size, size distribution, composition, and condition of the fat globules of the milk raw material,
- treating with a crosslinking enzyme,
- souring,
- optionally packaging the product.

Further, the sour milk products prepared by the process of the invention can be soured either before packaging the product or immediately after packaging. Especially viili-type products and set-type yogurts are soured in the package. Thus, according to an embodiment of the invention, the process comprises the following steps:
- physically modifying the particle size, size distribution, composition, and condition of the fat globules of the milk raw material,
- packaging,
- souring/allowing to sour and treating with a crosslinking enzyme in the package.

In the process of the invention, the heat-treatment may also be performed in several steps.

If necessary, the composition of the milk raw material used in the process may optionally be adjusted by standardizing its protein, fat, and/or lactose content. If desired/required, the milk raw material used in the process can optionally be heat-treated before the physical modification and/or after it.

Thus, according to an embodiment of the invention, the process comprises the following steps:
- adjusting the composition of the milk raw material, that is, standardizing its protein, fat, and/or lactose content,
- heat-treating the milk raw material,
- physically modifying the particle size, size distribution, composition, and condition of the fat globules of the milk raw material,
- heat-treating,
- souring,
- treating with a crosslinking enzyme,
- optionally packaging the obtained product.

In addition to souring, the process may also contain the use of rennet, and if necessary a subsequent heat treatment, that is, post-pasteurization.

Thus, according to an embodiment of the invention, the process comprises the following steps:
- standardizing, optionally, the composition of the milk raw material in terms of protein, fat, and/or lactose content,
- optionally heat-treating the milk raw material,
- physically modifying the particle size, size distribution, composition, and condition of the fat globules of the milk raw material,
- optionally heat-treating,
- souring,
- treating with a crosslinking enzyme,
- adding rennet or other enzymes,
- heat-treating,
- optionally packaging the obtained product.

Further, the process may also comprise the step of adding other raw materials after souring and treating with a crosslinking enzyme.

Thus, according to an embodiment of the invention, the process comprises the following steps:
- standardizing, optionally, the composition of the milk raw material in terms of protein, fat, and/or lactose content,
- optionally heat-treating the milk raw material,
- physically modifying the particle size, size distribution, composition, and condition of the fat globules of the milk raw material,
- optionally heat-treating,
- souring,
- treating with a crosslinking enzyme,
- optionally adding rennet,
- adding other raw materials,
- optionally heat-treating,
- optionally packaging the obtained product.

According to an embodiment of the invention, milk raw material standardized in terms of protein, fat, and/or carbohydrate contents is optionally heat-treated, the particle size, size distribution, composition and condition of its fat globules are physically modified, and it is modified with a crosslinking enzyme simultaneously with souring. According to a second embodiment of the invention, the formulation of the milk raw material is adjusted by standardizing its protein, fat and carbohydrate contents, the standardized milk raw material is heat-treated, the particle size, size distribution, composition and condition of the fat globules are modified by high-pressure homogenization at a temperature of 40 to 95°C and a pressure of 250 to 1000 bar, and modified with a crosslinking enzyme simultaneously with chemical souring.

In the process of the invention, the physical modification of the milk raw material can be done on the milk raw material or a part thereof in several steps with the same process or by combining different techniques in order to change the particle size, size distribution, composition and condition of the fat globules. The physical modification of the milk raw material or a part thereof can, thus, be done by high-pressure homogenization or microfiltration or by both. Thus, according to an embodiment of the invention, the process comprises the following steps:
- physically modifying the particle size, size distribution, composition, and condition of the fat globules of the milk raw material or a part thereof by using several techniques and/or several steps,
- optionally heat-treating,
- souring,
- treating with a crosslinking enzyme,
- optionally packaging the obtained product.

In one embodiment of the invention, the optional steps of the above described processes, such as standardizing the composition of the raw milk material, heat-treating, adding rennet or other enzymes and/or packing the product, are performed, i.e., they are not optional.

According to an embodiment of the invention, the milk raw material, which may be a standardized in terms of its protein, fat, and/or lactose content, is high-pressure homogenized, treated with a crosslinking enzyme, heat-treated and cooled, a separately high-pressure homogenized and/or micro-filtrated milk raw material high in fat content (30 to 50%, preferably 35%) is optionally added to it, a starter is added, the mixture is mixed and packaged.

In the process of the invention, the souring may be performed by adding a biological starter specific to each product (e.g. bulk starter or direct to vat starter DVI/DVS), a chemical starter, or organic or inorganic acids with or without adding rennet. For instance, the *Lactobacillus bulgaricus* and *Streptococcus thermophiles* strains are conventionally used in yogurt production. Examples of suitable organic acids include glucono-delta-lactone (GDL), calcium lactate, citric acid, and lactic acid. The used acid is preferably glucono-delta-lactone. In addition to lactic acid bacteria starters, viili-mould is also used in producing viili-type products.

The sour milk products prepared by the process of the invention can be soured either in a tank before packaging the product or immediately after packaging in a consumer or food service package.

Especially viili-type products and set-type yogurts are soured in the package.

The thickness of the texture is adjusted by altering the dosage of crosslinking enzymes. The product may be a "set type" (shearing and spoonable), drinkable (fresh), drinkable UHT, spoonable "yogurt-type" or powder (spray- or freeze-dried powder) product. Nutritionally, for instance, the essential amino acids in the proteins are in a well-absorbing form in the product.

Amino acids of animal and vegetable proteins may be crosslinked with enzymes, such as transglutaminase (EC 2.3.2.13). The covalent links formed in the enzyme process resist well different process conditions, such as heating and mixing. Of milk proteins, caseins and κ-casein in particular are the best substrate for transglutaminase. β-casein, too, is rich in glutamine and lysine that the enzyme links together. The used transglutaminase may be any transglutaminase enzyme used in the dairy industry, and it may originate from a microbe, yeast, mould, fish or mammal. The transglutaminase enzyme of an embodiment of the invention is microbial. There are several different commercially available transglutaminase enzyme preparations that are suitable for use in the process of the invention. These include Activa®YG (Ajinomoto, Japan) and Activa®MP (Ajinomoto, Japan). Optimum conditions depend on the used enzyme, and they can be obtained from the manufacturers of the commercial enzymes.

Other possible crosslinking and protein-modifying enzymes include laccase, tyrosinase, peroxidase, sulphydryl oxidase, glucose oxidase, protein glutaminase and in general other protein-modifying enzymes, such as chymosin and proteases. Tyrosinases (EC1.14.18.1) may originate from different vegetable, animal or fungal species, such as the *Trichoderma reesei* fungus. It is known that laccases (EC 1.10.3.2) originating from fungi or bacteria, such as the *Trametes hirsuta* fungus, hetero-crosslink carbohydrates and proteins. Tyrosinase and laccase preparations are also commercially available. Optimum conditions depend on the used enzyme, and they can be obtained from the manufacturers of the commercial enzymes. Said enzymes may be used either alone or in combination to achieve the desired result.

The process of the invention may also contain one or more further processing steps (e.g. mixing, separation, flavouring, cooling, packaging and/or product recovery specific to the product being prepared or dependent thereon), in which the soured milk raw material containing physically modified fat globules and treated with a crosslinking enzyme is processed.

In the process of the invention, the heat treatment(s) is/are performed in the manner known in the art. Examples of heat treatment processes useful for the process of the invention are pasteurization, high pasteurization, heating at a temperature lower than the pasteurization temperature for a sufficiently long time, thermisation, i.e., heating for at least 15 s at approximately 57 to 68°C, UHT, HT, and ESL treatments. In UHT, the raw material is heated at approximately 135 to 140°C for 2 to 4 s. HT ("short UHT treatment") is described in published patent application WO 2010085957. In ESL, the raw material is heated at approximately 127 to 135°C for 1 to 2 s. In pasteurization, the raw material is heated at approximately 70 to 72°C at least for 15 s, and in high pasteurization, the raw material is heated at approximately 95°C at least for 5 min. Heat treatment may also be a combination of different techniques.

According to the invention, raw milk (unseparated and unpasteurized milk, raw milk), which had been high-pressure-treated at a pressure of 400 bar and transglutaminase-treated during souring, was, depending on the storage time, 65 to 35% thicker than yogurt which had been merely high-pressure-treated at a pressure of 400 bar. Milks containing 0.4% and 1.0% fat and prepared in a corresponding manner, which were high-pressure-treated at a pressure of 400 bar and transglutaminase-treated during souring, were twice as thick as a control product that was merely high-pressure-treated.

Further, the viscosity of a yogurt prepared according to the invention and high-pressure-treated at a pressure of 400 bar and transglutaminase-treated during souring, was at one week 30% thicker than that of a yogurt homogenized at a pressure of 200 bar and transglutaminase-treated during souring, and 60% thicker than that of a yogurt homogenized at a pressure of 200 bar but not transglutaminase-treated.

According to the invention, the same effect can be produced by high-pressure homogenizing only the fat portion and combining it with non-homogenized fat-free milk or fat-free milk homogenized conventionally at low pressures, and treating the thus obtained milk with transglutaminase.

The texture of the products prepared with the process of the present invention remained homogeneous and whey did not separate during cold storage (+4°C, 3 weeks) or storage at room temperature (20 to 25°C, several weeks). An example of an advantageous embodiment of the process of the invention is the preparation of a yogurt having a protein content of 3.4 to 3.5. The viscosity of the fat (4.1%) yogurt of the invention was at one week 30% thicker and at three weeks 65% thicker than that of the control yogurt. The viscosity of the low-fat (1%) yogurt of the invention was 100% thicker than that of the control yogurt during the entire 3-week storage time. The viscosity of an even lower-fat (0.4%) yogurt of the invention was at one day 85%, at 1 to 2 weeks 50% and at 3 weeks 30% thicker than that of the control yogurt.

In addition, it was surprisingly found that the same effect can be produced by high-pressure homogenizing only the fat portion and combining it with non-homogenized fat-free milk or fat-free milk homogenized conventionally at low pressures, and treating the thus obtained milk with transglutaminase. An example of an advantageous embodiment of the invention is a yogurt that was prepared using the described process and raw materials: the fat portion (fat 35% and protein 2%) was high-pressure homogenized at a pressure of 1000 bar and at a temperature of 60°C, whipping cream/double cream and non-homogenized fat-free milk were combined to obtain a fat content of 1.5% and protein content of 3.5 for the raw material, it was pasteurized and cooled to 42°C, and TG enzyme 0.6 U/g (protein) and starter was added. The mixture was allowed to acidify until its pH was 4.5, it was then mixed, cooled to 20°C and packaged.

The method of the invention is simple and suitable for large-scale production.

According to an embodiment of the invention, the process is a component manufacture process, in which milk components having different fat and protein contents are combined only just before packaging.

The process of the present invention may be applied to both batch and continuous production. The method of the invention is preferably carried out as a batch process.

The invention also relates to a soured milk product that contains physically modified milk raw material fat globules, i.e. micro/nano particles of the milk raw material fat and has been produced by means of a crosslinking enzyme. According to an embodiment of the invention, the size of the fat globules of the soured milk-based product is less than 1 µm, preferably from about 20 nm to about 1 µm, more preferably from about 100 nm to 1 µm. Further, according to a second embodiment of the invention, the average particle size or medium size of the fat globules in the soured milk-based product is less than about 1 µm, preferably from about 200 nm to about 500 nm, and more preferably from about 300 nm to about 400 nm. According to a second embodiment of the invention, the size of the fat globules in the soured milk-based product is less than 1 µm, preferably from about 20 nm to about 1 µm, more preferably from about 100 nm to 1 µm, and the medium size is less than about 1 µm, preferably from about 200 nm to about 500 nm, and more preferably from about 300 nm to about 400 nm.

The following examples describe the performance of the invention, but without limiting the invention to said product embodiments.

### Example 1

### Yogurt produced of raw milk (400 bar and TG treatment)

The test yogurt of the invention was produced of raw milk (fat 4.1% and protein 3.4%) that was high-pressure homogenized at a temperature of 60°C and a pressure of 400 bar. After this, the milk was pasteurized and cooled to a temperature of 42°C. A TG enzyme (Activa®MP) 0.6 U/g (protein) and starter were added. The mixture was allowed to acidify until its pH was 4.5. The mixture was mixed and cooled to 20°C and packaged. After this, the mixtures were transferred to a cold room for cooling to a temperature of 5°C.

The control yogurt was prepared in the same manner except that no TG enzyme was added with the starter.

The viscosity of the yogurt of the invention was at one week 30% thicker and at three weeks 65% thicker than that of the control yogurt.

### Example 2

### Yogurt produced of milk containing 1.0% fat (400 bar and TG treatment)

The test yogurt of the invention was produced of milk (fat 1.0% and protein 3.5%) that was high-pressure homogenized at a temperature of 60°C and a pressure of 400 bar. After this, the milk was pasteurized and cooled to a temperature of 42°C. A TG enzyme (Activa®MP) 0.6 U/g (protein) and starter were added. The mixture was allowed to sour until its pH was 4.5. The mixture was mixed and cooled to 20°C and packaged. After this, the mixtures were transferred to a cold room for cooling to a temperature of 5°C.

The control yogurt was prepared in the same manner except that no TG enzyme was added with the starter.

The viscosity of the yogurt of the invention was 100% thicker than that of the control yogurt during the entire 3-week storage time.

### Example 3

### Yogurt produced of milk containing 0.4% fat (400 bar and TG treatment)

The test yogurt of the invention was produced of milk (fat 0.4% and protein 3.5%) that was high-pressure homogenized at a temperature of 60°C and a pressure of 400 bar. After this, the milk was pasteurized and cooled to a temperature of 42°C. A TG enzyme (Activa®MP) 0.6 U/g (protein) and starter were added. The mixture was allowed to acidify until its pH was 4.5. The mixture was mixed and cooled to 20°C and packaged. After this, the mixtures were transferred to a cold room for cooling to a temperature of 5°C.

The control yogurt was prepared in the same manner except that no TG enzyme was added with the starter.

The viscosity of the yogurt of the invention was at one day 85%, at 1 to 2 weeks 50% and at 3 weeks 30% thicker than that of the control yogurt.

### Example 4

### The difference between the yogurt of the invention produced of milk containing 0.4% fat (400 bar and TG treatment) and control yogurt 1 (200 bar) and control yogurt 2 (200 bar + TG treatment)

The test yogurt of the invention was produced of milk (fat 0.4% and protein 3.5%) that was high-pressure homogenized at a temperature of 60°C and a pressure of 400 bar. After this, the milk was pasteurized and cooled to a temperature of 42°C. A TG enzyme (Activa®MP) 0.6 U/g (protein) and starter were added. The mixture was allowed to sour until its pH was 4.5. The mixture was mixed and cooled to 20°C and packaged. After this, the mixtures were transferred to a cold room for cooling to a temperature of 5°C.

Control yogurt 1 was prepared in the same manner as the test yogurt of the invention except that no TG enzyme was added with the starter and the milk was homogenized at a pressure of 200 bar.

Control yogurt 2 was prepared in the same manner as the test yogurt of the invention except that the milk was homogenized at a pressure of 200 bar.

The viscosity of the yogurt of the invention was at one week 60% thicker than that of control yogurt 1, and control yogurt 2 was at one week 30% thicker than control yogurt 1.

### Example 5

### Separate homogenization of the fat portion (1000 bar) and yogurt produced of milk containing 1.5% fat (TG treatment)

The fat portion of the test yogurt of the invention (fat 35% and protein 2%) was high-pressure homogenized at a pressure of 1000 bar and a temperature of 60°C. After this, double cream and non-homogenized fat-free milk were combined to produce a fat content of 1.5% and protein content of 3.5% for the raw material. After this, the milk was pasteurized and cooled to a temperature of 42°C. A TG enzyme (Activa®MP) 0.6 U/g (protein) and starter were added. The mixture was allowed to acidify until its pH was 4.5. The mixture was mixed and cooled to 20°C and packaged. After this, the mixtures were transferred to a cold room for cooling to a temperature of 5°C.

The control yogurt was prepared in the same manner as the test yogurt of the invention except that no TG enzyme was added with the starter.

The viscosity of the yogurt of the invention was at one day and at three weeks 50% thicker than that of the control yogurt.

### Example 6

### Microfiltration of cream

Cream was filtered with Tetra Alcross MFS-1 microfiltration equipment with a ceramic, tube-type filtration membrane. The targeted top end pressure of the feed was approximately 4 bar. The pressure difference between the permeate and retentate side was approximately 0.4 bar.

Table 2 shows the total surface areas and average particle diameters of the unfiltered raw cream and microfiltered permeate and retentate calculated with the Malvern program.

**Table 2. Total surface areas and average particle diameters calculated with the Malvern program.**

| *Sample* | *Specific surface area* ***m²*/*g*** | *D[4,3] **µm*** |
|---|---|---|
| Raw cream | 1.67 | 4.167 |
| Permeate | 2.85 | 2.497 |
| Retentate | 1.79 | 3.851 |

### Illustrative Example 7 not falling within the scope of claim 1

### Yogurt produced using microfiltration permeate

Three yogurts were prepared, in which the fat content was adjusted to 2.5% and in which
a) non-homogenized fat-free milk was standardized with non-homogenized cream and the thus obtained milk raw material mixture (fat content 2.5%) was homogenized at a pressure of 140/20 bar (control),
b) the milk raw material (fat content 2.5%) was obtained by adding to non-homogenized fat-free milk non-homogenized cream permeate that was obtained from raw cream by microfiltration, and
c) the milk raw material (fat content 2.5%) was obtained by adding to non-homogenized fat-free milk non-homogenized cream permeate (control).

It was found that in the non-homogenized standardized raw material milk (c), fat rose to the top, whereas in the homogenized (a) or cream permeate-containing (b) milk raw material, no phase separation occurred. The milk raw materials were pasteurized at a temperature of 90°C for 5 min and soured with a yogurt starter at 42°C/pH 4.5. The gelated yogurt was mixed. The yogurt with a conventionally homogenized fat (average particle size around 0.5 µm) was the thickest (200 to 400 mPas). The texture of this yogurt thickened further during storage. The yogurt without homogenization (average particle size of fat around 4 µm) was around 100 mPas in thickness when fresh and 200 mPas after three weeks of storage. With the cream permeate (average particle size of fat 1.8 µm), the thicknesses of the yogurt were 100 and 125 mPas. The yogurt with the cream permeate was the most stable in texture, its viscosity did not change during storage. Whey did not separate from any sample.

## Claims

1. A process for the manufacture of a sour milk product, **characterised in that** the fat portion of a milk raw material is physically modified by high-pressure homogenization at a pressure of 250-1000 bar and/or microfiltration and that the milk raw material containing physically modified fat globules is treated with a crosslinking enzyme and soured.

2. The process as claimed in claim 1, **characterised in that** the milk raw material containing physically modified fat globules is obtained by physically modifying the particle size and size distribution of the fat globules of the milk raw material.

3. The process as claimed in claim 1, **characterised in that** the milk raw material containing physically modified fat globules is obtained by physically modifying the fat portion of the milk raw material and by combining it with non-homogenized or conventionally homogenized fat-free milk.

4. The process as claimed in claim 1, **characterised in that** the milk raw material containing physically modified fat globules is obtained by combining native and/or physically modified fat globules with non-homogenized or conventionally homogenized fat-free milk.

5. The process as claimed in any one of claims 1 to 4, **characterised in that** the treatment with a crosslinking enzyme is done before souring, simultaneously with souring, or after souring.

6. The process as claimed in claim 1, **characterised in that** the process comprises the following steps:
- physically modifying the particle size, size distribution, composition, and condition of the fat globules of the milk raw material,
- souring,
- treating with a crosslinking enzyme,
- optionally packaging the obtained product.

7. The process as claimed in claim 1, **characterised in that** the process comprises the following steps:
- physically modifying the particle size, size distribution, composition, and condition of the fat globules of the milk raw material,
- packaging,
- souring and treating with a crosslinking enzyme in the package.

8. The process as claimed in claim 6, **characterised in that** the process comprises the following steps:
- optionally adjusting the composition of the milk raw material,
- optionally heat-treating the milk raw material,
- physically modifying the particle size, size distribution, composition, and condition of the fat globules of the milk raw material,
- optionally heat-treating,
- souring,
- treating with a crosslinking enzyme,
- optionally adding rennet,
- optionally heat-treating,
- optionally adding other raw materials,
- optionally packaging the obtained product.

9. The process as claimed in any one of claims 1 to 8, **characterised in that** the souring is done biologically by adding a starter, product inoculation starter, chemical starter, organic acids or inorganic acids with or without adding rennet.

10. The process as claimed in any one of the preceding claims, **characterised in that** the process is a component manufacture process, in which milk components having different fat and protein contents are combined only just before packaging.

11. The process as claimed in any one of the preceding claims, **char**- **acterised** in that the preparation process is either a continuous or batch process.

12. The process as claimed in any one of the preceding claims, **char**- **acterised** in that souring is performed either in a tank before the product is packaged or immediately after packaging in a consumer or food service package.

13. A sour milk product, **characterised in that** it is prepared by a process of any one of claims 1 to 12.

14. A sour milk product, **characterised in that** the size of the fat globules in the product is from 20 nm to 1 µm.

15. The product as claimed in claim 14, **characterised in that** the medium size of the fat globules is less than 1 µm, preferably from 200 nm to 500 nm.

## Patentansprüche

1. Verfahren zur Herstellung eines Sauermilchprodukts, **dadurch gekennzeichnet, dass** der Fettanteil eines Milchrohmaterials durch Hochdruckhomogenisierung bei einem Druck von 250-1000 bar und/oder Mikrofiltration physikalisch modifiziert wird und dass das Milchrohmaterial, das physikalisch modifizierte Fettkügelchen enthält, mit einem vernetzenden Enzym behandelt und gesäuert wird.

2. Verfahren wie in Anspruch 1 beansprucht, **dadurch gekennzeichnet, dass** das Milchrohmaterial, das physikalisch modifizierte Fettkügelchen enthält, durch physikalisches Modifizieren der Partikelgröße und Größenverteilung der Fettkügelchen des Milchrohmaterials erhalten wird.

3. Verfahren wie in Anspruch 1 beansprucht, **dadurch gekennzeichnet, dass** das Milchrohmaterial, das physikalisch modifizierte Fettkügelchen enthält, durch physikalisches Modifizieren des Fettanteils des Milchrohmaterials und durch Kombinieren desselben mit nicht homogenisierter oder konventionell homogenisierter fettfreier Milch erhalten wird.

4. Verfahren wie in Anspruch 1 beansprucht, **dadurch gekennzeichnet, dass** das Milchrohmaterial, das physikalisch modifizierte Fettkügelchen enthält, durch Kombinieren von nativen und/oder physikalisch modifizierten Fettkügelchen mit nicht homogenisierter oder konventionell homogenisierter fettfreier Milch erhalten wird.

5. Verfahren wie in einem der Ansprüche 1 bis 4 beansprucht, **dadurch gekennzeichnet, dass** die Behandlung mit einem vernetzenden Enzym vor dem Säuern, gleichzeitig mit dem Säuern oder nach dem Säuern erfolgt.

6. Verfahren wie in Anspruch 1 beansprucht, **dadurch gekennzeichnet, dass** das Verfahren die folgenden Schritte umfasst:
- physikalisches Modifizieren der Partikelgröße, Größenverteilung, Zusammensetzung und des Zustands der Fettkügelchen des Milchrohmaterials,
- Säuern,
- Behandeln mit einem vernetzenden Enzym,
- gegebenenfalls Verpacken des erhaltenen Produkts.

7. Verfahren wie in Anspruch 1 beansprucht, **dadurch gekennzeichnet, dass** das Verfahren die folgenden Schritte umfasst:
- physikalisches Modifizieren der Partikelgröße, Größenverteilung, Zusammensetzung und des Zustands der Fettkügelchen des Milchrohmaterials,
- Verpacken,
- Säuern und Behandeln mit einem vernetzenden Enzym in der Packung.

8. Verfahren wie in Anspruch 6 beansprucht, **dadurch gekennzeichnet, dass** das Verfahren die folgenden Schritte umfasst:
- gegebenenfalls Einstellen der Zusammensetzung des Milchrohmaterials,
- gegebenenfalls Wärmebehandeln des Milchrohmaterials,
- physikalisches Modifizieren der Partikelgröße, Größenverteilung, Zusammensetzung und des Zustands der Fettkügelchen des Milchrohmaterials,
- gegebenenfalls Wärmebehandeln,
- Säuern,
- Behandeln mit einem vernetzenden Enzym,
- gegebenenfalls Zugeben von Labferment,
- gegebenenfalls Wärmebehandeln,
- gegebenenfalls Zugeben von anderen Rohmaterialien,
- gegebenenfalls Verpacken des erhaltenen Produkts

9. Verfahren wie in einem der Ansprüche 1 bis 8 beansprucht, **dadurch gekennzeichnet, dass** das Säuern biologisch durch Zugeben von einem Starter, einer Starterkultur, einem chemischen Starter, von organischen Säuren oder anorganischen Säuren mit oder ohne Zugabe von Labferment erfolgt.

10. Verfahren wie in einem der vorangehenden Ansprüche beansprucht, **dadurch gekennzeichnet, dass** das Verfahren ein Komponentenherstellungsverfahren ist, in welchem Milchkomponenten mit unterschiedlichen Fett- und Proteingehalten nur unmittelbar vor dem Verpacken kombiniert werden.

11. Verfahren wie in einem der vorangehenden Ansprüche beansprucht, **dadurch gekennzeichnet, dass** das Herstellungsverfahren entweder ein kontinuierliches oder diskontinuierliches Verfahren ist.

12. Verfahren wie in einem der vorangehenden Ansprüche beansprucht, **dadurch gekennzeichnet, dass** das Säuern entweder in einem Behälter durchgeführt wird, bevor das Produkt verpackt wird, oder unmittelbar nach dem Verpacken in einer Verbraucherverpackung oder einem Verpflegungspaket durchgeführt wird.

13. Sauermilchprodukt, **dadurch gekennzeichnet, dass** es durch ein Verfahren nach einem der Ansprüche 1 bis 12 hergestellt wird.

14. Sauermilchprodukt, **dadurch gekennzeichnet, dass** die Größe der Fettkügelchen in dem Produkt 20 nm bis 1 µm beträgt.

15. Produkt wie in Anspruch 14 beansprucht, **dadurch gekennzeichnet, dass** die mittlere Größe der Fettkügelchen weniger als 1 µm, vorzugsweise von 200 nm bis 500 nm beträgt.

## Revendications

1. Procédé de fabrication d'un produit laitier acidifié, **caractérisé en ce que** la portion matière grasse d'une matière première laitière est physiquement modifiée par homogénéisation à haute pression à une pression de 250 à 1 000 bars et/ou microfiltration et **en ce que** la matière première laitière contenant les globules de matière grasse physiquement modifiée est traitée avec une enzyme de réticulation et acidifiée.

2. Procédé tel que revendiqué selon la revendication 1, **caractérisé en ce que** la matière première laitière contenant des globules de matière grasse physiquement modifiée est obtenue en modifiant physiquement la taille des particules et la distribution des tailles des globules de matière grasse de la matière première laitière.

3. Procédé tel que revendiqué selon la revendication 1, **caractérisé en ce que** la matière première laitière contenant des globules de matière grasse physiquement modifiée est obtenue en modifiant physiquement la portion matière grasse de la matière première laitière et en la combinant avec du lait sans matière grasse non homogénéisé ou homogénéisé de manière conventionnel.

4. Procédé tel que revendiqué selon la revendication 1, **caractérisé en ce que** la matière première laitière contenant des globules de matière grasse physiquement modifiée est obtenue en combinant des globules de matière grasse d'origine naturelle et/ou physiquement modifiée avec du lait sans matière grasse non homogénéisé ou homogénéisé de manière conventionnel.

5. Procédé tel que revendiqué selon l'une quelconque des revendications 1 à 4, **caractérisé en ce que** le traitement avec une enzyme de réticulation est effectué avant l'acidification, simultanément à l'acidification, ou après l'acidification.

6. Procédé tel que revendiqué selon la revendication 1, **caractérisé en ce que** le procédé comprend les étapes suivantes :
- de modification physiquement de la taille des particules, de la distribution des tailles, de la composition, et de l'état des globules de matière grasse de la matière première laitière,
- d'acidification,
- de traitement avec une enzyme de réticulation,
- éventuellement de conditionnement du produit obtenu.

7. Procédé tel que revendiqué selon la revendication 1, **caractérisé en ce que** le procédé comprend les étapes suivantes :
- de modification physiquement de la taille des particules, de la distribution des tailles, de la composition, et de l'état des globules de matière grasse de la matière première laitière,
- d'emballage,
- d'acidification et de traitement avec une enzyme de réticulation dans l'emballage.

8. Procédé tel que revendiqué selon la revendication 6, **caractérisé en ce que** le procédé comprend les étapes suivantes :
- éventuellement d'ajustement de la composition de la matière première laitière,
- éventuellement de traitement thermique de la matière première laitière,
- de modification physiquement de la taille des particules, de la distribution des tailles, de la composition, et de l'état des globules de matière grasse de la matière première laitière,
- éventuellement de traitement thermique,
- d'acidification,
- de traitement avec une enzyme de réticulation,
- éventuellement d'addition de présure,
- éventuellement de traitement thermique,
- éventuellement d'addition d'autres matières premières,
- éventuellement de conditionnement du produit obtenu.

9. Procédé tel que revendiqué selon l'une quelconque des revendications 1 à 8, **caractérisé en ce que** l'acidification est effectué biologiquement par l'addition d'un starter, d'un starter d'inoculation de produit, d'un produit starter chimique, d'acides organiques ou d'acides inorganiques avec ou sans addition de présure.

10. Procédé tel que revendiqué selon l'une quelconque des revendications précédentes, **caractérisé en ce que** le procédé est un procédé de fabrication de constituant, dans lequel les constituants laitiers ayant différentes teneurs en matière grasse et en protéine sont associés uniquement juste avant le conditionnement.

11. Procédé tel que revendiqué selon l'une quelconque des revendications précédentes, **caractérisé en ce que** le procédé de préparation est soit un procédé continu soit discontinu.

12. Procédé tel que revendiqué selon l'une quelconque des revendications précédentes, **caractérisé en ce que** l'acidification est effectué soit dans un tank avant que le produit soit emballé ou soit immédiatement après l'emballage dans un consommateur ou dans un emballage de restauration.

13. Produit laitier acidifié, **caractérisé en ce qu'**il est préparé selon un procédé selon l'une quelconque des revendications 1 à 12.

14. Produit laitier acidifié, **caractérisé en ce que** la taille des globules de matière grasse dans le produit est de 20 nm à 1 µm.

15. Produit tel que revendiqué selon la revendication 14, **caractérisé en ce que** la taille moyenne des globules de matière grasse est inférieure à 1 µm, préférablement de 200 nm à 500 nm.
